(19) Europäisches Patentamt
European Patent Office
Office européen des brevets

(11) Veröffentlichungsnummer : **0 043 013**
**B1**

# (12) EUROPÄISCHE PATENTSCHRIFT

(45) Veröffentlichungstag der Patentschrift : **01.06.83**

(51) Int. Cl.³ : **C 07 D277/68**

(21) Anmeldenummer : **81104488.2**

(22) Anmeldetag : **11.06.81**

(54) **Verfahren zur Herstellung von 2-Chlor-benzthiazolen.**

(30) Priorität : **21.06.80 DE 3023227**

(43) Veröffentlichungstag der Anmeldung : **06.01.82 Patentblatt 82/01**

(45) Bekanntmachung des Hinweises auf die Patenterteilung : **01.06.83 Patentblatt 83/22**

(84) Benannte Vertragsstaaten : **BE CH DE FR GB IT LI**

(56) Entgegenhaltungen : **GB A 966 496**

(73) Patentinhaber : **HOECHST AKTIENGESELLSCHAFT**
**Postfach 80 03 20**
**D-6230 Frankfurt/Main 80 (DE)**

(72) Erfinder : **Papenfuhs, Theodor, Dr.**
**Heinrich-Bleicher-Strasse 40**
**D-6000 Frankfurt am Main 50 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 043 013 B1

# Verfahren zur Herstellung von 2-Chlor-benzthiazolen

Die Erfindung betrifft ein neues Verfahren zur Herstellung von 2-Chlor-benzthiazolen.

2-Chlor-benzthiazole sind technisch wichtige Zwischenprodukte, die beispielsweise aus der belgischen Patentschrift 670 434 und der deutschen Offenlegungsschrift 29 24 712 bekannt sind. Die Synthese dieser Verbindungen ist technisch bisher nicht befriedigend gelöst. So stoßen die bekannten Verfahren beispielsweise auf Schwierigkeiten in der technischen Ausführbarkeit, liefern schlechte Ausbeuten oder sind mit ökologischen Problemen verbunden, oder aber die einzusetzenden Ausgangsverbindungen sind schlecht zugänglich. Bisher bekannte Synthesevorschläge basieren auf zwei prinzipiell unterschiedlichen Wegen : Der eine läuft über die Chlorierung von Benzthiazolen und ihren in 2-Stellung durch Merkapto- oder Hydroxygruppen substituierten Derivaten, der andere über eine Sandmeyer-Reaktion von 2-Amino-benzthiazolen durch Diazotierung und kupferkatalysierte, salzsaure Verkochung der so erhaltenen Diazoniumchloride.

Bei dem oben erwähnten Chlorierungsweg können beispielsweise die Benzthiazole und 2-Hydroxybenzthiazole nur mittels Phosphorpentachlorid in die 2-Chlor-benzthiazole übergeführt werden [vgl. Chem. Ber. *12*, 1127 (1879) und *13*, 9 (1880) ; J. Chem. Soc. *1925*, 1488 ; US-PS 1 878 699]. Zur Chlorierung von 2-Merkaptobenzthiazolen wurden Phosphorpentachlorid und/oder Phosphoroxichlorid (s. DE-PS 516 998 und US-PS 1 878 699), elementares Chlor [J. Am. Chem. Soc. *68*, 1666 (1946)], Phosgen (vgl. DE-PS 1 164 413 und GB-PS 913 910), Dischwefeldichlorid (s. CA-PS 321 452 und US-PS 2 043 948), Thionylchlorid (vgl. DE-AS 1 545 663) und Sulfurylchlorid (s. US-PS 2 469 697 ; GB-PS 646 045 ; DE-OS 2 138 829) vorgeschlagen. Diese Chlorierungsmethoden verlaufen zwar vorwiegend mit annehmbaren Ausbeuten, sind aber mit anderen Nachteilen verbunden. So sind die verwendeten Ausgangsverbindungen meist schlecht zugänglich, insbesondere, wenn es sich um kernsubstituierte Benzthiazolderivate handelt ; Chlorierungsmittel, wie Phosphorpentachlorid und Phosgen, sind technisch nur äußerst schwierig einsetzbar, und andere Chlorierungsmittel verlangen wegen ihrer hohen Abgaslast apparative Investitionen, die die Verfahren wirtschaftlich unergiebig machen. — Demgegenüber liefert der Syntheseweg über die Sandmeyer-Reaktion [vgl. Gazz. Chim. Ital. *92*, 1221 (1962) und *94*, 372 (1964)] ausnahmslos schlechte Ausbeuten, so daß eine technische Durchführung dieser Verfahrensweise trotz günstigerer Ökologie und besserer Zugänglichkeit der Ausgangsprodukte ausgeschlossen ist.

Es bestand somit ein erhebliches Interesse an einem allgemein anwendbaren, technisch realisierbaren und besseren Verfahren zur Herstellung von 2-Chlor-benzthiazolen.

Mit dieser Erfindung wurde nunmehr ein Verfahren zur Herstellung von 2-Chlor-benzthiazolen gefunden, das dadurch gekennzeichnet ist, daß man ein 2-Hydrazino-benzthiazol mit der mindest stöchiometrischen Menge Thionylchlorid, gegebenenfalls in Gegenwart eines inerten Löse- oder Verdünnungsmittels, umsetzt.

Die Reaktion läuft nach folgendem Schema :

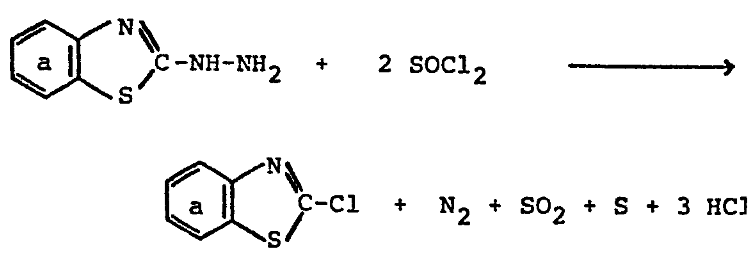

(der Benzolkern a kann substituiert sein).

Diese erfindungsgemäße Reaktion verläuft mit sehr hohen Ausbeuten. Die bei der Reaktion entstehenden sauren Abgase lassen sich durch eine kombinierte Gaswäsche, beispielsweise zuerst mit Wasser und anschließend mit einer Alkalilauge, wie Natronlauge, nahezu quantitativ in wäßrige Salzsäure und Alkalibisulfit-Lösung überführen, die sich technisch verwerten lassen. Die sauren Abgase belasten somit weder die Abluft noch das Abwasser.

Bei dem erfindungsgemäßen Verfahren wird das Thionylchlorid in zumindest stöchiometrischer Menge, d. h. 2 Mol Thionylchlorid pro Mol 2-Hydrazinobenzthiazol-Verbindung, eingesetzt. Meist verwendet man jedoch einen Überschuß an Thionylchlorid, der nach obenhin nicht begrenzt sein muß, den man allerdings aus wirtschaftlichen Erwägungen so niedrig hält, daß das Reaktionsgemisch noch ausreichend rührbar ist, sofern ein Lösemittel nicht als Reaktionsmedium eingesetzt wird. In der Regel wird deshalb ein 0,1- bis 2,0-molarer, bevorzugt 0,25- bis 1,0-molarer Überschuß an Thionylchlorid verwendet. Die Umsetzung kann deshalb bevorzugt in Abwesenheit von Lösemitteln durchgeführt werden. Sofern man Löse- oder Verdünnungsmittel als Reaktionsmedium einsetzt, so dürfen diese bei der oxidativ ablaufenden Chlorierungsreaktion nicht mit den Reaktionskomponenten reagieren.

Die Reaktionstemperatur kann im Bereich von etwa 10 °C bis zur Siedetemperatur des Thionylchlorids (79 °C) oder gegebenenfalls des als Reaktionsmedium eingesetzten Löse- oder Verdünnungsmittels

liegen ; die Reaktion läuft praktisch spontan ab. Bevorzugt liegt die Reaktionstemperatur zwischen 35 und 65 °C.

Das erfindungsgemäße Verfahren wird besonders vorteilhaft in der Weise durchgeführt, daß man die 2-Hydrazino-benzthiazol-Verbindung unter Rühren in Thionylchlorid einträgt, das zuvor auf etwa 35-65 °C erwärmt wurde und gegebenenfalls mit einem Löse- oder Verdünnungsmittel versetzt ist ; hierbei kann das 2-Hydrazino-benzthiazol ebenfalls in dem entsprechenden Löse- oder Verdünnungsmittel gelöst oder suspendiert sein. Bei dieser exotherm ablaufenden Chlorierungsreaktion kann die frei werdende Reaktionswärme beispielsweise durch die entweichenden Gase abgeführt werden. Nach dem Eintragen des 2-Hydrazinobenzthiazols kann die Reaktionsmischung noch einige Zeit nachgerührt werden, insbesondere um die restlichen sauren Abgase auszutreiben.

Die Isolierung der erfindungsgemäß hergestellten 2-Chlor-benzthiazol-Verbindung kann durch fraktionierte Destillation des Reaktionsansatzes erfolgen, bei dem nach eventuellem Vorlauf von überschüssig angewandtem Thionylchlorid und gegebenenfalls Löse- oder Verdünnungsmittel die 2-Chlorbenzthiazol-Verbindung rein überdestilliert. Der in der Reaktion gebildete Schwefel verbleibt, — bei Destillationstemperaturen oberhalb von 100 °C in schmelzflüssiger Form —, im Destillationssumpf. Er kann in üblicher Weise aufgearbeitet oder einer anderen Verwertung zugeführt werden. — Bei der Herstellung von nicht-destillierbaren oder höher schmelzenden 2-Chlor-benzthiazol-Verbindungen wird der Reaktionsansatz vorteilhaft in Wasser eingerührt, um überschüssiges Thionylchlorid zu hydrolisieren. Wurde die erfindungsgemäße Reaktion, wie bevorzugt, in Abwesenheit eines Löse- oder Verdünnungsmittels durchgeführt, so läßt sich die in Wasser ausfallende 2-Chlorbenzthiazolverbindung mittels einer Wasserdampfdestillation rein isolieren, wobei sie aus dem abgekühlten Destillat durch Filtration leicht abgetrennt werden kann ; man kann aber auch hierbei so verfahren, daß der schwefelhaltige Niederschlag des Hydrolysats abfiltriert wird und dieses durch Schwefel verunreinigte 2-Chlor-benzthiazol direkt der weiteren Umsetzung zu anderen Endprodukten zuführt ; diese Arbeitsweise bietet sich an, wenn der enthaltene Schwefel bei den gewünschten Folgereaktionen mit dem 2-Chlor-benzthiazol nicht stört und während der weiteren Umsetzung leicht, beispielsweise durch Filtration, abgetrennt werden kann. Da dies für die meisten Folgeumsetzungen zutrifft, stellt diese Aufarbeitungsmöglichkeit des chlorierungsansatzes eine besonders vorteilhafte Methode dar. Die mit dem Schwefel verunreinigte, aus dem Hydrolysat erhältliche 2-Chlor-benzthiazol-Verbindung kann jedoch auch von dem Schwefel in der Weise abgetrennt werden, daß man dieses Gemisch, gegebenenfalls nach dessen vorheriger Trocknung, einer Kalt-Extraktion zuführt, wobei man solche Lösemittel verwendet, die die 2-Chlor-benzthiazol-Verbindung lösen, den Schwefel hingegen nicht oder nur in äußerst geringen Mengen ; solche Lösemittel sind z. B. Aceton, Alkanole, Chloraliphaten, Benzol, Dialkylbenzole und Halogenbenzole. Aus dem erhaltenen Extrakt läßt sich die 2-Chlor-benzthiazol-Verbindung durch Abdampfen des Extraktionsmittels in reiner Form gewinnen.

Ist bei der erfindungsgemäßen Chlorierungsreaktion ein Löse- oder Verdünnungsmittel mitverwendet worden, so kann man nach Beendigung der Reaktion den ungelösten Schwefel von dem Reaktionsansatz durch Filtration abtrennen ; der Reaktionsansatz kann auch bei Verwendung von Löse- oder Verdünnungsmitteln der oben erwähnten Hydrolyse unterworfen werden, wobei man nach einer Klärfiltration ein Zweiphasengemisch erhält, aus welchem man die Lösung der 2-Chlor-benzthiazol-Verbindung abtrennt und diese durch Abdestillieren des Lösungsmittels isoliert. Gegebenenfalls kann die organische Lösung dieser reinen 2-Chlor-benzthiazol-Verbindung vorteilhaft auch direkt in die gewünschte Folgereaktion der Weiterverarbeitung der 2-Chlor-benzthiazol-Verbindung eingesetzt werden.

Die als Ausgangsverbindungen dienenden 2-Hydrazino-benzthiazol-Verbindungen lassen sich in hohen Ausbeuten, beispielsweise gemäß den Angaben der US-PS 3 937 714, herstellen ; die hierzu dienenden 2-Amino-benzthiazol-Verbindungen sind leicht zugänglich und lassen sich besonders vorteilhaft gemäß den Angaben der DE-PS 28 01 991 in sehr hohen Ausbeuten synthetisieren. Das erfindungsgemäße Verfahren geht deshalb von leicht zugänglichen und in hoher Ausbeute erhältlichen Ausgangsverbindungen aus und führt selbst zu sehr hohen, teilweise quantitativen Ausbeuten. Die erfindungsgemäß hergestellten Verbindungen werden in hoher Reinheit erhalten. Zudem läßt sich das erfindungsgemäße Verfahren in einfacher Weise technisch und ohne ökologische Entsorgungsschwierigkeiten durchführen, so daß es insgesamt einen neuen Syntheseweg zur Herstellung von 2-Chlorbenzthiazol-Verbindungen darstellt, der in der Technik problemlos durchgeführt werden kann.

Das erfindungsgemäße Verfahren dient bevorzugt zur Herstellung von 2-Chlor-benzthiazol-Verbindungen der allgemeinen Formel (1)

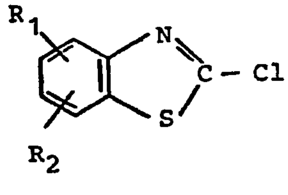

(1)

in welcher $R_1$ und $R_2$ gleich oder verschieden voneinander sind und jedes ein Wasserstoffatom, eine

Alkylgruppe, bevorzugt niedere Alkylgruppe, eine Alkoxygruppe, bevorzugt niedere Alkoxygruppe, ein Halogenatom, bevorzugt ein Fluor- oder Chloratom, eine Aminogruppe, die durch niederes Alkyl und/oder Phenyl substituiert sein kann, eine Carboxygruppe, eine Carbonsäurealkylestergruppe, bevorzugt eine niedere Carbonsäurealkylestergruppe, eine Carbonsäurephenylestergruppe, die Trifluormethyl-, Cyan-, eine durch niederes Alkyl und/oder Phenyl gegebenenfalls substituierte Carbamoylgruppe, einen Arylrest, wie vorzugsweise einen gegebenenfalls substituierten Phenylrest, eine Acylgruppe, Acylaminogruppe oder Nitrogruppe bedeuten, wobei unter « Acyl » bevorzugt der Acylrest der Benzoesäure oder einer niederen aliphatischen Carbonsäure zu verstehen ist. Zur Herstellung dieser bevorzugten Verbindungen der allgemeinen Formel (1) geht man von entsprechenden 2-Hydrazino-benzthiazol-Verbindungen der allgemeinen Formel (2)

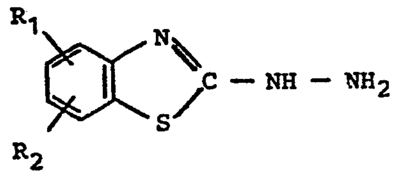

(2)

aus, in welcher $R_1$ und $R_2$ die obengenannten Bedeutungen haben.

Geht man von 2-Hydrazino-benzthiazol-Verbindungen, bspw. entsprechend der allgemeinen Formel (2), aus, in welchen der Benzolkern einen Substituenten enthält, der eine gegenüber Thionylchlorid reagierbare Carbonylgruppierung besitzt, wie beispielsweise die Carboxygruppe oder eine Carbonsäureestergruppe, so erhält man zusätzlich die entsprechende Carbonsäurechloridgruppe als neuen Substituenten in dem Benzolkern ; in gleicher Weise wird die Carbamoylgruppe als Substituent durch das Thionylchlorid in die Cyangruppe übergeführt. Ist der Benzolkern durch eine unsubstituierte Aminogruppe substituiert, so wird diese mit Thionylchlorid zwar intermediär in die Thionylaminogruppe übergeführt, jedoch kann die freie Aminogruppe bei der hydrolytischen Aufarbeitung wieder zurückgebildet werden.

Bevorzugt ist insbesondere das erfindungsgemäße Verfahren zur Herstellung der Verbindungen der allgemeinen Formel (1), in welcher $R_1$ und $R_2$ gleich oder verschieden voneinander sind und jedes ein Wasserstoffatom, eine niedere Alkylgruppe, wie Methyl- oder Äthylgruppe, eine niedere Alkoxygruppe, wie Methoxy- oder Äthoxygruppe, ein Halogenatom, wie bevorzugt ein Chloratom, eine Nitrogruppe, eine Cyan- oder Aminogruppe bedeuten, wobei man von solchen Verbindungen der allgemeinen Formel (2) ausgeht, in welchen $R_1$ und $R_2$ die eben angegebenen Bedeutungen besitzen.

Die nachfolgenden Beispiele dienen zur Erläuterung der Erfindung. Die Teile sind Gewichtsteile, die Prozentangaben beziehen sich auf Gewichtsprozente, sofern nicht anders vermerkt. Gewichtsteile stehen im Verhältnis zu Volumenteilen wie Kilogramm zu Liter.

## Beispiel 1

Im Verlauf von 3 Stunden werden 82,5 Teile 2-Hydrazino-benzthiazol gleichmäßig unter Rühren in 166,6 Teile Thionylchlorid von einer Temperatur von 55 °C eingetragen. Die entweichenden sauren Gase werden in 2 hintereinander geschalteten Absorptionsanlagen (Waschflaschen) absorbiert, von denen die erste mit 540 Teilen Wasser, die zweite mit 1 040 Teilen einer 10 %igen wäßrigen Natronlauge gefüllt ist. Während des Eintragens des 2-Hydrazino-benzthiazols wird die Chlorierungstemperatur von 55 °C durch leichtes Erwärmen mittels Außenheizung aufrechterhalten. Der Reaktionsansatz wird danach 30 Minuten bei 55 °C nachgerührt. Man destilliert sodann bei Normaldruck eventuell noch vorhandenes Thionylchlorid (10-20 Teile) ab und destilliert anschließend unter vermindertem Druck. Bei einer Siedetemperatur von 127 °C bei 13,5 mbar werden 77,5 Teile Destillat erhalten, die analytisch und chromatographisch reines 2-Chlor-benzthiazol sind. Die Ausbeute entspricht somit 91,5 % d. Th.

Die Absorptionsvorlagen (Waschflaschen) enthalten nach der Umsetzung 730 Teile einer etwa 20 %igen wäßrigen Salzsäure bzw. 1 210 Teile einer etwa 23 %igen wäßrigen Natriumbisulfitlösung, die natriumchlorid-frei ist. — Der Destillationsrückstand (etwa 20 Teile) kann in schmelzflüssiger Form entleert und in üblicher Weise entsorgt werden.

## Beispiel 2

Man verfährt in der im Beispiel 1 angegebenen Verfahrensweise, setzt jedoch anstelle von 2-Hydrazino-benzthiazol 98,5 Teile 5-Methyl-2-hydrazino-benzthiazol als Ausgangsverbindung ein.

Nach Beendigung der Reaktion wird die Reaktionsmischung mit 200 Teilen Tetrachlormethan verdünnt, der ausgefallene Schwefel (16,5 Teile) durch Filtration abgetrennt und das Filtrat fraktioniert destilliert. Nach Abdestillieren des Lösemittels und einer geringen Menge an Thionylchlorid bei Normaldruck erhält man bei 132 °C und 13,5 mbar das 5-Methyl-2-chlor-benzthiazol in einer Ausbeute

von 94,9 % d. Th. Das Produkt ist analysenrein und besitzt einen Schmelzpunkt von 42 °C.

Ersetzt man das Tetrachlormethan durch Dichlormethan oder Toluol und arbeitet sonst in der hier angegebenen Weise, so erhält man das 5-Methyl-2-chlor-benzthiazol in gleich guter Ausbeute und Reinheit.

Beispiel 3

Man verfährt in der in Beispiel 1 angegebenen Verfahrensweise der Chlorierung, setzt jedoch 89,5 Teile 7-Methyl-2-hydrazino-benzthiazol mit 142,8 Teilen Thionylchlorid um und wählt die Reaktionstemperatur bei 45 °C.

Nach Beendigung der Reaktion wird der Ansatz in 500 Teile kaltes Wasser einlaufen lassen, in welchem überschüssiges Thionylchlorid hydrolysiert ; das Hydrolysat wird mit Natronlauge auf einen pH-Wert von 7 eingestellt und sodann einer Wasserdampfdestillation unterworfen. Man erhält ein zweiphasiges Destillat, deren untere organische Schicht analysenreines 7-Methyl-2-chlorbenzthiazol ist ; die erhaltene Menge entspricht einer Ausbeute von 91,5 % d. Th. Es zeigt einen Erstarrungspunkt von + 1,5 °C und ist bei 1,3 mbar bei einer Kondensationstemperatur von 95 °C unzersetzt destillierbar.

Beispiel 4

199,5 Teile 6-Chlor-2-hydrazino-benzthiazol werden gemäß der im Beispiel 1 angegebenen Verfahrensweise bei einer Reaktionstemperatur von 65 °C mit 357 Teilen Thionylchlorid umgesetzt. Nach Beendigung der Reaktion wird der Ansatz in 1 500 Teile kaltes Wasser einlaufen lassen ; nach der Hydrolyse wird der ausgefallene Niederschlag abfiltriert und bei 80 °C getrocknet. Man erhält 214 Teile eines Produktes, das, wie chromatographisch nachweisbar, neben Schwefel lediglich das gewünschte 2,6-Dichlorbenzthiazol enthält und somit frei von organischen Verunreinigungen ist. Dieses Produkt kann für die meisten Folgereaktionen, in die dieses 2-Chlor-benzthiazol-Derivat zwecks Weiterverarbeitung eingesetzt werden kann, wie beispielsweise Umsetzungen des 2-ständigen Chlors mit Aminen, Alkoholen, Phenolen, Merkaptanen oder Hydrazinen, ohne weitere Reinigung verwendet werden, da der in diesem Produkt enthaltene Schwefel diese Reaktionen nicht stört und im Reaktionsgang, beispielsweise durch Klärfiltration, leicht abtrennbar ist.

Zur Isolierung des reinen 2,6-Dichlor-benzthiazols wird das oben erhaltene Reaktionsprodukt, vorteilhaft ohne vorherige Trocknung, mit 1 000 Teilen Aceton verrührt ; ungelöster Schwefel wird abfiltriert, und das Acetonfiltrat wird entweder mit 3 000 Teilen Wasser versetzt, wobei das 2,6-Dichlorbenzthiazol ausfällt und abfiltriert werden kann, oder eingedampft. Man erhält in beiden Fällen 180,5 Teile (entsprechend 88,5 % d. Th.) 2,6-Dichlor-benzthiazol mit einem Schmelzpunkt von 99 °C.

Ersetzt man das Aceton durch die entsprechende Menge Äthanol und arbeitet sonst in der hier angegebenen Weise, so erhält man das 2,6 Dichlor-benzthiazol in praktisch gleich hoher Ausbeute und gleich guter Reinheit.

Beispiel 5

Eine Mischung aus 105 Teilen 4-Nitro-2-hydrazino-benzthiazol und 350 Teilen Chlorbenzol wird unter Rühren auf 80 °C erwärmt. Innerhalb von 4 Stunden tropft man 130 Teile Thionylchlorid hinzu. Die sauren Abgase werden gemäß den Angaben des Beispieles 1 absorbiert. Nach Zugabe des Thionylchlorides rührt man noch 1 Stunde bei 90-95 °C, sodann noch 30 Minuten bei 120 °C nach, um Reste von nicht umgesetztem Thionylchlorid auszutreiben, das in den Absorptionsanlagen hydrolysiert und absorbiert wird. Den Reaktionsansatz kühlt man sodann auf eine Temperatur von 10 °C ab, trennt den ausgefallenen Schwefel ab und unterwirft das chlorbenzolische Filtrat einer Wasserdampfdestillation, bei welcher nur das Chlorbenzol übergeht. Der Destillationssumpf wird nach Abkühlen filtriert, der Filterrückstand mit Wasser gewaschen und getrocknet. Man erhält 98,7 Teile 4-Nitro-2-chlor-benzthiazol mit einem Schmelzpunkt von 169 °C entsprechend einer Ausbeute von 92,0 % d. Th.

Ersetzt man das Chlorbenzol durch die gleiche Menge eines technischen Xylol-Gemisches, so erhält man ein identisches Ergebnis.

Beispiel 6

20,9 Teile 2-Hydrazino-benzthiazol-5-carbonsäure werden mit 80 Teilen Tetrachloräthan verrührt und auf 50 °C erwärmt. Bei dieser Temperatur tropft man innerhalb von 2 Stunden 40 Teile Thionylchlorid hinzu (die sauren Abgase werden gemäß den Angaben des Beispieles 1 absorbiert). Anschließend rührt man noch 2 Stunden bei 70 °C, sodann 2 Stunden unter Rückfluß nach und kühlt sodann den Reaktionsansatz auf 0 °C ab. Der Schwefel wird abfiltriert, das Filtrat unter reduziertem Druck auf 30 Teile eingeengt. Sodann gibt man 120 Teile Petroläther hinzu, aus dem das 2-Chlor-benzthiazol-5-carbonsäurechlorid in farblosen Nadeln ausfällt. Man saugt es ab, wäscht es mit Petroläther und trocknet es. Man erhält 19,9 Teile dieser Verbindung, die einen Schmelzpunkt von 131 °C besitzt, entsprechend einer Ausbeute von 85,9 % d. Th.

Beispiel 7

36,0 Teile 5-Amino-2-hydrazino-benzthiazol werden gemäß den Verfahrensangaben des Beispieles 1, jedoch unter Anwendung einer Reaktionstemperatur von 35-40 °C, mit 95,0 Teilen Thionylchlorid umgesetzt. Die erhaltene Schmelze des 2-Chlor-benzthiazol-5-thionylamins wird sodann in 350 Teile Wasser einfließen lassen und darin hydrolysiert. Man rührt eine Stunde bei 25-30 °C nach, trennt die saure Lösung nach Zusatz von 2 Teilen Aktivkohle vom ausgefallenen Schwefel ab und stellt das Filtrat unter Rühren durch Zutropfen von Natronlauge auf einen pH-Wert zwischen 8 und 8,5. Hierbei fällt das 5-Amino-2-chlor-benzthiazol farblos aus ; es wird abfiltriert, mit Wasser gewaschen und getrocknet. Es ist analysenrein, besitzt einen Schmelzpunkt von 147-148 °C und wird in einer Ausbeute von 30,3 Teilen entsprechend 82,0 % d. Th. erhalten.

Beispiel 8

20,8 Teile 2-Hydrazino-benzthiazol-6-carbonamid werden im Verlauf von 3 Stunden gleichmäßig unter Rühren in 50 Teile heißes Thionylchlorid eingetragen, sodaß die Umsetzung unter Rückflußtemperatur erfolgt. Der so erhaltene Reaktionsansatz wird sodann noch 3 Stunden unter Rückfluß nachgerührt, anschließend in ein Gemisch aus 100 Teilen Wasser und 100 Teilen Benzol einlaufen lassen und darin 1 Stunde gerührt. Der ausgefallene Schwefel wird nach Zusatz von einem Teil Aktivkohle abfiltriert. Aus dem zweiphasigen Filtrat wird die Benzolphase abgetrennt und über Natriumsulfat getrocknet ; das Benzol wird sodann unter reduziertem Druck abdestilliert. Man erhält als Rückstand 17,5 Teile 6-Cyan-2-chlor-benzthiazol mit einem Schmelzpunkt von 73 °C entsprechend 90,0 % d. Th.

Ersetzt man hier das Benzol durch Chloroform, so erhält man ein identisches Ergebnis.

Beispiele 9 bis 28

Verfährt man in erfindungsgemäßer Weise zur Herstellung von 2-Chlor-benzthiazol-Verbindungen, beispielsweise in analoger Weise, wie in den obigen Ausführungsbeispielen 1-5 beschrieben, und geht hierbei von 2-Hydrazino-benzthiazol-Verbindungen gemäß der allgemeinen Formel (2) mit den in den nachfolgenden Tabellenbeispielen angegebenen Substituenten $R_1$ und $R_2$ aus, so erhält man die entsprechenden 2-Chlor-benzthiazol-Verbindungen gemäß der allgemeinen Formel (1) mit den in den Tabellenbeispielen angegebenen Substituenten $R_1$ und $R_2$ mit den dort ebenfalls angegebenen Ausbeuten und Schmelzpunkten bzw. Siedepunkten.

| Bsp. | Verbindung Formel (1) $R_1$ | entsprechend bzw. (2) $R_2$ | Ausbeute an (1) (% d.Th.) | Smp. (°C) | Sdp. (°C/mbar) |
|---|---|---|---|---|---|
| 9 | 4-Methyl | H | 90,7 | 48 | |
| 10 | 6-Methyl | H | 92,1 | 49 | |
| 11 | 4-Methyl | 6-Methyl | 87,7 | 33 - 34 | |
| 12 | 4-Methyl | 7-Methyl | 82,5 | 38 - 39 | |
| 13 | 4-Methoxy | H | 90,3 | 66 | |
| 14 | 5-Methoxy | H | 80,0 | 50 | |
| 15 | 6-Methoxy | H | 93,1 | 54 | 130/9,3 |
| 16 | 6-Äthoxy | H | 94,0 | 67 | 146/10,7 |
| 17 | 4-Chlor | H | 91,5 | 97 | |
| 18 | 5-Chlor | H | 86,0 | 69 | |
| 19 | 7-Chlor | H | 79,2 | 52 | |
| 20 | 4-Methyl | 6-Chlor | 83,8 | 69,5 - 70 | |
| 21 | 4-Methyl | 7-Chlor | 75,8 | 52 - 53 | |
| 22 | 5-Methoxy | 6-Chlor | 92,0 | 126 | |
| 23 | 4-Chlor | 6-Chlor | 95,4 | 106 | 118/0,7 |
| 24 | 6-Brom | H | 92,4 | 88 | 158/24 |
| 25 | 5-Trifluor-methyl | H | 86,0 | 62 | |

Fortsetzung

| Bsp. | Verbindung entsprechend Formel (1) bzw. (2) $R_1$ | $R_2$ | Ausbeute an (1) (% d.Th.) | Smp. (°C) | Sdp. (°C/mbar) |
|---|---|---|---|---|---|
| 26 | 5-Nitro | H | 94,8 | 111 | |
| 27 | 6-Nitro | H | 93,9 | 191 | |
| 28 | 7-Nitro | H | 84,3 | 114 | |

**Anspruch**

Verfahren zur Herstellung von 2-Chlor-benzthiazolen, dadurch gekennzeichnet, daß man ein 2-Hydrazinobenzthiazol mit der mindest stöchiometrischen Menge Thionylchlorid, gegebenenfalls in Gegenwart eines inerten Löse- oder Verdünnungsmittels, umsetzt.

**Claim**

Process for the preparation of 2-chloro-benzthiazoles, characterized in that a 2-hydrazino-benzthiazole is reacted with the at least stoichiometric amount of thionyl chloride, optionally in the presence of an inert solvent or diluent.

**Revendication**

Procédé pour préparer des chloro-2 benzothiazoles, caractérisé en ce qu'on fait réagir un hydrazino-2 benzothiazole avec une quantité de chlorure de thionyle au moins égale à la quantité stœchiométrique, éventuellement en présence d'un solvant ou diluant inerte.